# EUROPEAN PATENT APPLICATION

(11) **EP 2 778 179 A2**
(43) Date of publication of application: **17.09.2014**
(21) Application number: 12837216.6
(22) Date of filing: 27.09.2012
(51) Int. Cl.: C08B 37/08, C08L 5/08, A61L 27/20, A61L 33/08

(54) **CHITOSAN AND/OR CHITIN COMPOSITE HAVING REINFORCED PHYSICAL PROPERTIES AND USE THEREOF**

(30) Priority: 27.09.2011 KR 20110097691
(71) Applicant: Postech Academy-Industry Foundation, Pohang-si, Gyeongsangbuk-do 790-784 (KR)
(72) Inventor: HWANG, Dong Soo, Pohang-si Gyeongsangbuk-do 790-390 (KR); OH, Dong Yeop, Busan 601-010 (KR); KIM, Sang Sik, Pohang-si Gyeongsangbuk-do 791-753 (KR); CHA, Hyung Joon, Pohang-si Gyeongsangbuk-do 790-751 (KR)
(74) Representative: Potter Clarkson LLP
(86) International application number: PCT/KR2012/007822
(87) International publication number: WO 2013/048144

(57) **Abstract**

The present invention relates to a composite including chitosan and/or chitin and a catechol-based compound, an organic reinforcing material composition including the composite, a product manufactured by using the organic reinforcing material composition, and a method for preparing a chitosan and/or chitin composite with improved strength, including the step of adding the catechol-based compound to chitosan and/or chitin. The chitosan and/or chitin composite including the catechol-based compound is advantageous in that it is able to maintain high strength in a wet-swollen state by improving the problem of strength reduction due to moisture, compared to the composites containing no catechol-based compound.

## Description

### [TECHNICAL FIELD]

The present invention relates to a chitosan and/or chitin composite including one or more compounds selected from the group consisting of catechol, dopamine, DOPA, and methyl catechol, an organic reinforcing material composition including the composite, a product manufactured by using the organic reinforcing material composition, and a method for preparing the chitosan and/or chitin composite with improved strength, including the step of adding one or more compounds selected from the group consisting of catechol, dopamine, DOPA, and methyl catechol to chitosan and/or chitin.

### [BACKGROUND ART]

Chitin and chitosan are tasteless and odorless natural polysaccharide polymers, chitin is a polysaccharide polymer material that is composed of repeating units of N-acetyl-D-glucosamine, and chitosan is a polysaccharide polymer material that is composed of repeating units produced by the removal of acetyl groups from chitin.

Since chitin and chitosan are natural compounds, they have excellent biocompatibility and meet all requirements for functional foods. Furthermore, they are evaluated as highly valuable multi-functional materials in a wide range of applications, including medical fields such as artificial skin, operation suture, artificial dialysis membrane, various medical auxiliaries, etc., industrial fields such as textile, cosmetics, household products, waste water treatment, film for photography, dye, paper manufacture, biodegradable plastics, etc., agricultural fields such as soil conditioner, fertilizer, pollution free pesticide, feed, etc., removal of radioactive contamination, liquid crystal, or ion exchange membrane, etc.

However, strength of the conventional chitin/chitosan is rapidly reduced under wet conditions or in water, and therefore, there are difficulties in their commercial applications, for example, artificial tendon or artificial ligament under wet conditions such as blood or lymph fluid in the body. According to a recent report for the cause of this phenomenon, moisture functions as a plasticizer to reduce strength and glass transition temperature of chitin and chitosan [Carbohydrate Polymers 83 (2011) 947]. In order to solve this problem, there is a need for a technology to develop a biomaterial source that has improved strength under wet conditions and thus functions as, for example, artificial tendon or artificial ligament under wet conditions while maintaining multi-functional availability.

### [SUMMARY OF THE INVENTION]

The present inventors found that a biomaterial (organic reinforcing material) such as artificial tendon or artificial ligament with improved strength under wet conditions or in water can be developed by adding one or more compounds selected from the group consisting of catechol, dopamine, DOPA, and methyl catechol to chitosan and/or chitin, thereby completing the present invention.

Accordingly, one aspect of the present invention provides a chitosan and/or chitin composite including one or more compounds selected from the group consisting of catechol, dopamine, DOPA, and methyl catechol.

Another aspect of the present invention provides an organic reinforcing material composition including the composite, and use of the composite in the preparation of the organic reinforcing material composition.

Still another aspect of the present invention provides a product that is manufactured by using the organic reinforcing material composition.

Still another aspect of the present invention provides a method for preparing the chitosan and/or chitin composite with improved strength, including the step of adding one or more compounds selected from the group consisting of catechol, dopamine, DOPA, and methyl catechol to chitosan and/or chitin.

### [DETAILED DESCRIPTION]

As described above, the present invention provides a chitosan and/or chitin composite including one or more compounds selected from the group consisting of catechol, dopamine, DOPA (dihydroxyphenylalanine), and methyl catechol, an organic reinforcing material composition including the composite, a product manufactured by using the organic reinforcing material composition, and a method for preparing the chitosan and/or chitin composite with improved strength, including the step of adding one or more compounds selected from the group consisting of catechol, dopamine, DOPA, and methyl catechol to chitosan and/or chitin. The chitosan and/or chitin composite including one or more compounds selected from the group consisting of catechol, dopamine, DOPA, and methyl catechol is advantageous in that the problem of strength reduction due to moisture is remarkably improved, compared to a composite not including one or more compounds selected from the group consisting of catechol, dopamine, DOPA, and methyl catechol, and thus high strength can be maintained under wet conditions.

In particular, the chitin and/or chitosan composite can be provided as a material for artificial tendon and artificial ligament. It is preferable that artificial tendon and artificial ligament have such high strength that they function to connect bones and muscles when tendon and ligament are broken, and also have such high biocompatibility that they are absorbed into the body when new tissues are generated or they are no longer needed.

Chitin and chitosan, as abundant and eco-friendly resources, have many advantages of biodegradability, anti-viral and wound-healing properties, etc., and thus are suitable as biomaterials such as artificial tendon and artificial ligament. However, artificial tendon and artificial ligament are applied under wet conditions such as blood or lymph fluid in the body, and the strength of the conventional chitin and chitosan is rapidly reduced under wet conditions or in water. Therefore, there are difficulties in their commercialization as biomaterials that must maintain strength under wet conditions, such as artificial tendon or artificial ligament.

As described above, according to the preliminary test results of the present inventors, chitin and chitosan were found to have the problem of remarkably low strength (modulus) in contact with water or under wet conditions. When strength (modulus) of chitin/chitosan was compared between a completely dried sample and a sample immersed in a 0.15 M phosphate buffered saline (pH 7.4) aqueous solution for a day, tensile strength (Young's modulus) of the hydrated sample of chitin and chitosan film was greatly reduced to 10% of tensile strength of the completely dried sample. In this regard, it was reported that moisture functions as a plasticizer to reduce strength and glass transition temperature of chitin and chitosan [Carbohydrate Polymers 83 (2011)].

Meanwhile, it is considered that melanin pigment is produced by cross-linking reaction between catechol composites such as DOPA. It is accepted that as cross-linking density of DOPA increases and hydrophobic melanin increases, dehydration occurs, leading to reinforcement of physical properties of the material (Andersen, S. O. et al, Nature 251, 507 (1974)).

Accordingly, the present inventors found that a material with improved strength can be developed by adding one or more compounds selected from the group consisting of catechol, dopamine, DOPA, and methyl catechol to chitosan and/or chitin, thereby completing the present invention.

First, the present invention provides a composite of chitosan, chitin, or a mixture thereof, including chitosan, chitin, or the mixture thereof and one or more compounds selected from the group consisting of catechol, dopamine, DOPA, and methyl catechol (e.g., 3-methyl catechol), in which chitosan, chitin, or the mixture thereof is crosslinked to one or more compounds selected from the group consisting of catechol, dopamine, DOPA, and methyl catechol via a covalent or non-covalent bond. This composite is characterized in that its mechanical properties such as tensile strength (young's modulus) are remarkably improved when it is swollen in water, compared to chitosan, chitin, or the mixture thereof alone. Owing to such improvement in mechanical properties, the composite can be preferably applied to artificial ligaments, artificial tendons, or other uses which are required to have strong mechanical properties and low water absorption property under wet environments (humid conditions).

Such strength-improving effect is achieved by adding one or more compounds selected from the group consisting of catechol, dopamine, DOPA, and methyl catechol to chitosan, chitin, or the mixture thereof, and the degree of the strength-improving effect increases depending on the addition amount of one or more compounds selected from the group consisting of catechol, dopamine, DOPA, and methyl catechol. Therefore, the addition amount of one or more compounds selected from the group consisting of catechol, dopamine, DOPA, and methyl catechol is not limited to a particular range, but it is preferably added in an amount of 0.1 to 30% by weight, or 1 to 30% by weight, or 4 to 30% by weight, or 15 to 30% by weight based on the weight (100% by weight) of chitosan, chitin, or the mixture thereof, in order to obtain the desired strength-improving effect while maintaining intrinsic bioavailability of chitosan, chitin, or the mixture thereof.

In the present invention, a molecular weight of chitosan or chitin is not particularly limited, but it may be in the range of 5 to 500 kDa. In the composite of the present invention, chitosan and chitin may be included singly, or in the form of the mixture thereof.

The composite maintains excellent physical properties even after it is completely immersed in distilled water for 3 hours to be wet and swollen (see Tables 2 and 3).

The composite further includes one or more compounds selected from the group consisting of catechol, dopamine, DOPA, and methyl catechol (e.g., 3-methyl catechol), in addition to chitosan and/or chitin, and therefore, crosslinking and dehydration reactions by oxidation of one or more compounds selected from the group consisting of catechol, dopamine, DOPA, and methyl catechol prevents reduction in mechanical properties due to moisture, thereby maintaining excellent physical properties.

Further, the composite includes a relatively large amount of melanin because production of hydrophobic melanin is increased by oxidation of one or more compounds selected from the group consisting of catechol, dopamine, DOPA, and methyl catechol. Consequently, dehydration due to melanin helps to reinforce physical properties of the material. The content of melanin in the composite may be about 50% by weight or more, for example, about 50% by weight to about 99% by weight or about 70% by weight to about 99% by weight, specifically about 75% by weight to about 98% by weight, and more specifically 80% by weight to about 98% by weight as measured by a hydrogen peroxide degradation method (Moses, D and J. H Waite, Journal of the biological chemistry, 2006, Vol. 281, Issue 46, 34826-34832), but is not limited thereto.

This description can be confirmed by the results of tensile strength improvement and water absorption reduction which will be demonstrated in the following Examples. The water absorption can be measured by a typical method, for example, EWC (equilibrium water content). The water absorption was tested after immersing a sample in a 0.15 M phosphate buffered saline (pH 7.4) aqueous solution for a day, and changes in the weight were measured using a scale with a minimal resolution of 0.0001. EWC can be calculated by the following Equation: 100X(Wₜ-W₀)/Wₜ(W0: the dry weight of sample, Wt: the weight of sample no longer absorbing water).

As such, since oxidation of one or more compounds selected from the group consisting of catechol, dopamine, DOPA, and methyl catechol helps to maintain and reinforce physical properties of the composite, the composite was found to have remarkably increased physical properties such as tensile strength in a wet-swollen state when it further includes a compound as an oxidant, for example, sodium periodate, hydrogen peroxide, sodium iodate (sodium iodate), and/or sodium hydroxide (see Table 3). Therefore, the composite of the present invention may further include one or more selected from the group consisting of sodium periodate, hydrogen peroxide, sodium iodate, and sodium hydroxide (NaOH). The amount of one or more selected from the group consisting of sodium periodate, hydrogen peroxide, and sodium hydroxide further included may be 5 to 15% by weight, specifically 8 to 12% by weight, based on one or more compounds selected from the group consisting of catechol, dopamine, DOPA, and methyl catechol.

Further, the strength of the chitosan or chitin composite in the wet state can be further improved by heat treatment (annealing) (see Table 3). The heat treatment can be carried out at 80 to 120°C, specifically, at 90 to 110°C under vacuum for 6 to 12 hours.

The chitosan or chitin composite may have young's modulus of about 500 Mpa or more, for example, 500 to 10000 Mpa, or 500 to 5000 Mpa at a relative humidity of about 40 to 50%, and young's modulus of about 180 Mpa or more, specifically about 280 Mpa or more, more specifically 300 Mpa or more, for example, 300 to 5000 Mpa, or 300 to 3000 Mpa at a relative humidity of about 90 to 100%. Therefore, it is possible to apply the composite to biomaterials such as artificial tendon and artificial ligament which are required to have good strength under wet conditions of the body.

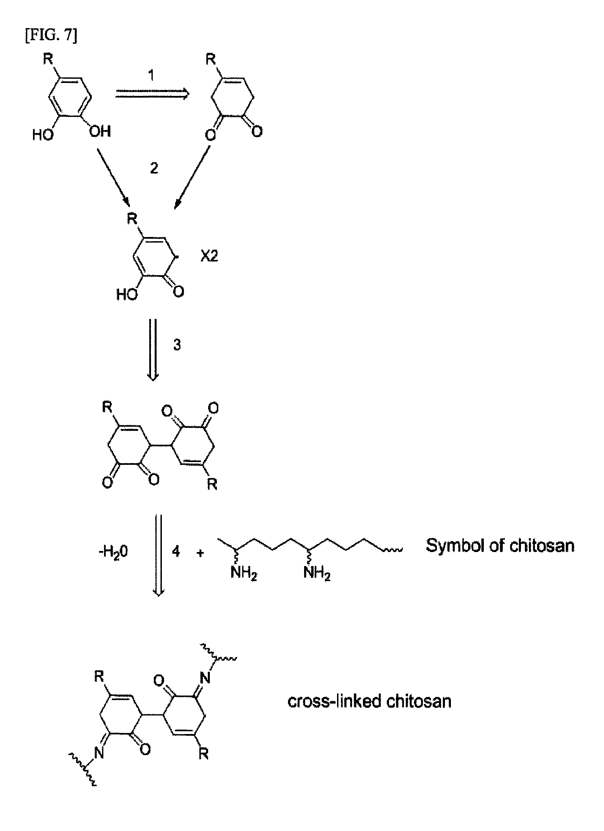

The composite according to the present invention may have a structure, in which one or more compounds selected from the group consisting of catechol, dopamine, DOPA, and methyl catechol are crosslinked to the amine group of chitosan or chitin via a covalent bond (in particular, in the case of using sodium periodate or hydrogen peroxide), or a non-covalent bond (e.g., cation-π bond) (see FIG. 7). FIG. 7 is a schematic diagram showing the reactions that may occur between the amine group of chitosan and dopamine or catechol upon addition of sodium periodate (indicated by oxidant). In FIG. 7, Reactions 1 to 3 are the reactions which can be accelerated by addition of sodium periodate, and Reaction 4 is the reaction which occurs at elevated temperatures under vacuum, resulting in water formation (INTEGR. COMP. BIOL., 42:1172-1180 (2002) Adhesion a la Moule1, J. H. Waite).

Another embodiment of the present invention provides an organic reinforcing material composition including the composite with improved physical properties such as mechanical strength. More particularly, provided is an organic reinforcing material composition, including the composite of chitosan, chitin, or the mixture thereof containing chitosan, chitin, or the mixture thereof and one or more compound selected from the group consisting of catechol, dopamine, DOPA, and methyl catechol (e.g., 3-methyl catechol), in which chitosan, chitin, or the mixture thereof is crosslinked to one or more compounds selected from the group consisting of catechol, dopamine, DOPA, and methyl catechol via a covalent or non-covalent bond. Still another embodiment of the present invention provides use of the composite of chitosan, chitin, or the mixture thereof in the preparation of the organic reinforcing material composition, in which the composite contains chitosan, chitin, or the mixture thereof and one or more compound selected from the group consisting of catechol, dopamine, DOPA, and methyl catechol (e.g., 3-methyl catechol), and chitosan, chitin, or the mixture thereof is crosslinked to one or more compounds selected from the group consisting of catechol, dopamine, DOPA, and methyl catechol via a covalent or non-covalent bond. A detailed description of the composite is the same as described above. The organic reinforcing material composition may be in the form of film, filament, or non-woven fabric, but is not limited thereto. It may be all material compositions required to have strength.

Still another embodiment provides a product that is manufactured by using the organic reinforcing material composition including the composite of chitosan, chitin, or the mixture thereof containing chitosan, chitin, or the mixture thereof and one or more compound selected from the group consisting of catechol, dopamine, DOPA, and methyl catechol (e.g., 3-methyl catechol), in which chitosan, chitin, or the mixture thereof is crosslinked to one or more compounds selected from the group consisting of catechol, dopamine, DOPA, and methyl catechol via a covalent or non-covalent bond. The product may be all reinforcing material products including biomaterials which are applied to the body and are required to have strength.

Still another embodiment provides use of the composite of chitosan, chitin, or the mixture thereof in the preparation of the reinforcing material product, in which the composite contains chitosan, chitin, or the mixture thereof and one or more compound selected from the group consisting of catechol, dopamine, DOPA, and methyl catechol (e.g., 3-methyl catechol), and chitosan, chitin, or the mixture thereof is crosslinked to one or more compounds selected from the group consisting of catechol, dopamine, DOPA, and methyl catechol via a covalent or non-covalent bond.

The reinforcing material product may be, for example, artificial ligament, artificial tendon, artificial dental materials (e.g., artificial Sharpey's fiber, artificial periodental ligament, etc.), artificial skin, operation suture, artificial dialysis membrane, various medical auxiliaries, textile for clothes, tire cord (reinforcing fabric material inside rubber to increase durability, driving performance, and safety of tire) or the like.

Still another embodiment provides a method for improving strength of chitosan and/or chitin or a method for preparing chitosan and/or chitin with improved strength. The method may include the steps of 1) dissolving chitosan, chitin, or the mixture thereof in water, an ionic solvent or a mixture thereof; and 2) adding one or more selected from the group consisting of catechol, dopamine, DOPA, and methyl catechol to the solution thus obtained.

The ionic solvent means all ionic liquids capable of dissolving chitosan and/or chitin, and examples thereof may include acetic acid or acetic acid aqueous solution, DMAc (dimethylacetamide)/LiCl (LiCl in DMF (dimethylformamide), chitosan or chitin can be dissolved therein up to a weight ratio of 5 to 10%), ethylmethylimidazolium acetate or the like, and specifically, 0.1 to 5 M acetic acid aqueous solution.

The addition amount of one or more compounds selected from the group consisting of catechol, dopamine, DOPA, and methyl catechol may be 0.1 to 30% by weight, or 1 to 30% by weight, or 4 to 30% by weight, or 15 to 30% by weight, based on the weight of chitosan, chitin, or the mixture thereof.

Further, the method may further include the step of 2-1) adding one or more selected from the group consisting of sodium periodate, hydrogen peroxide, sodium iodate, and sodium hydroxide, for example, in an amount of 5 to 15% by weight, preferably 8 to 12% by weight, based on one or more selected from the group consisting of catechol, dopamine, DOPA, and methyl catechol. Step 2-1) may be carried out before or after step 2) or concurrent with step 2).

The method may further include the heat-treatment step (step 3) after step 2) or 2-1). For example, the heat-treatment step 3) may be carried out by heat-treating the mixture obtained in step 2) or 2-1) (the mixture of chitosan and/or chitin and one or more selected from the group consisting of catechol, dopamine, DOPA, and methyl catechol; or the mixture of chitosan and/or chitin, one or more selected from the group consisting of catechol, dopamine, DOPA, and methyl catechol, and one or more selected from the group consisting of sodium periodate, hydrogen peroxide, and sodium hydroxide) at 80 to 120°C, preferably at 90 to 110°C under vacuum for 6 to 12 hours. Through such heat-treatment step, strength of the final product can be further improved.

Since the composite prepared by adding catechol, dopamine, or the mixture thereof to chitosan, chitin, or the mixture thereof in the present invention maintains the improved strength even after immersed in water, it can be applied to a variety of biomaterials such as artificial tendon, artificial ligament, artificial dental material. The use thereof is not limited thereto, and the composite can be usefully applied to various materials.

### [BRIEF DESCRIPTION OF DRAWINGS]

FIG. 1 shows an initial graph obtained during measurement of tensile strength and a calculation method of tensile strength;
FIGs. 2 and 3 show graphs obtained during tensile strength test of C15 vs C15_SP_annealing 70% (FIG. 2) and D15 vs D15_SP_annealing 70% (FIG. 3) at a relative humidity of 90 to 100%, respectively;
FIGs. 4a to 4e are graphs showing tensile strength, stiffness, and toughness of the chitosan composite containing crosslinked chitosan and DOPA in a dry state;
FIG. 5 is a graph showing tensile strength, stiffness, and toughness according to the contents of DOPA and oxidant in a wet-swollen state;

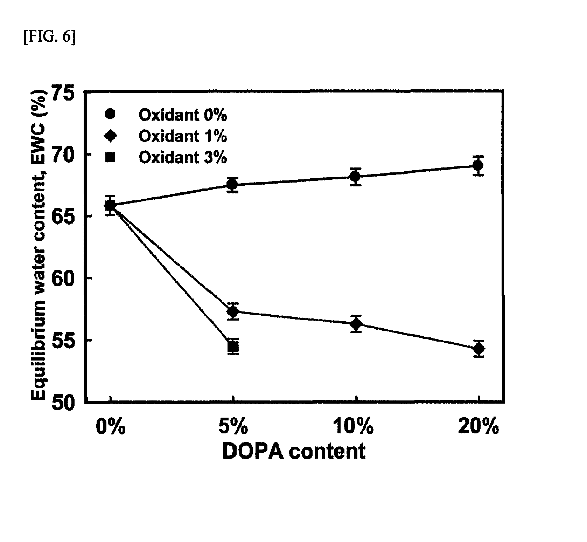

FIG. 6 is a graph showing EWC (Equilibrium Water Content) of the chitosan composite according to the content of DOPA;
FIG. 7 is a schematic diagram showing a covalent bond between chitin or chitosan and dopamine or catechol (addition of sodium periodate or heat treatment);
FIG. 8 shows comparison of static water contact angle between chitosan and chitosan composite;
FIG. 9 is a graph showing that mechanical properties are improved due to reduced water absorption of DOPA-crosslinked chitosan;
FIG. 10 shows static water contact angle (top) and EWC (bottom) of pure chitin and chitin composite containing dopamine;
FIG. 11 shows SEM images of the electron microscopic structures of a pure chitin film and a chitin composite film;
FIG. 12 is a graph showing a crystalline structure of a chitin composite which contains dopamine in an amount of 10% by weight, in which the black color (the lowest in the graph) indicates native chitin, the red color (the middle in the graph) indicates a chitin film, and the blue color (the top in the graph) indicates a dopamine-containing chitin composite film;
FIG. 13 is a graph showing cytotoxicity of the final chitin fiber on osteoblastic cell (MC3T3-e);
FIG. 14 is a graph showing the result of TGA (thermal gravimetric analysis) test of chitosan and chitosan composite;
FIG. 15 is a UV-IR spectrum at 300 nm to 700 nm of the chitin composite according to Example 7 or a sepia melanin solution degraded from the chitosan composite by a hydrogen peroxide degradation method; and
FIG. 16 is a melanin calibration curve obtained from absorbance of the sepia melanin solution of FIG. 15.

Hereinafter, the constitutions and effects of the present invention will be described in more detail with reference to Examples, Comparative Examples and Experimental Examples. However, these Examples, Comparative Examples and Experimental Examples are for illustrative purposes only, and the scope and the range of the present invention are not intended to be limited by these Examples.

### Example 1: Preparation of chitosan composite containing catechol or dopamine

After a 0.325 M acetic acid aqueous solution was prepared, 20 g of chitosan (High molecular weight, sigma-aldrich, Chitosan 419419-(Coarse ground flakes and powder) 800-2000 cP, 1 wt% in 1%(w/v) acetic acid, Brookfield (lit.), DDA: 80% or more) was dissolved in 980 g of the acetic acid aqueous solution under sonication at 40°C for 24 hours to prepare a chitosan/acetic acid aqueous solution. To 30 ml of the chitosan/acetic acid aqueous solution thus obtained, Catechol (99%(w/w), sigma-aldrich) and dopamine (99%(w/w) sigma-aldrich) were added at a mixing ratio satisfying the conditions as in the following Table 1, and dissolved, respectively.

**[Table 1]**

| Name of sample | Mixing ratio |
|---|---|
| Neat chitosan | chitosan/acetic acid aqueous solution |
| C01 | chitosan:catechol=100:1(g/g) |
| C04 | chitosan:catechol=100:4(g/g) |
| C07 | chitosan:catechol=100:7(g/g) |
| C15 | chitosan:catechol=100:15(g/g) |
| D15 | chitosan:dopamine=100:15(g/g) |

Sodium periodate was added to the samples C15 and D15 in an amount corresponding to 10% by weight of catechol or dopamine contained therein, and the mixtures obtained were designated as C15 SP and D15 SP, respectively.

Each 30 ml of the prepared samples was added to a petri dish of which entire bottom was coated with Teflon tape (a separate petri dish was used for each sample). Each petri dish prepared was dried in a convection oven at 40°C for 2 days to prepare freestanding films of about 0.1 mm. To completely remove the residual acetic acid and water, the obtained films were placed in a vacuum oven at 50°C overnight.

### Example 2: Preparation of chitin and chitin composite

Chitin (chitin from shrimp, Sigma-Aldrich) was dissolved in an ionic liquid (1-Ethyl-3-methylimidazolium acetate) to 10% by weight so as to prepare a chitin solution. Dopamine (99wt%, Sigma-Aldrich) was added to the chitin solution thus prepared in an amount of 0% by weight, 5% by weight, or 10% by weight. To completely dissolve the solute, the chitin solution or chitin/dopamine solution was placed at 100°C for 6 hours. These two solutions completely dissolved were poured into a mold, and treated at 150°C for 2 hours for oxidation and crosslinking reactions of dopamine by heat. Thereafter, the two solutions were left at room temperature overnight to be cooled, and they became gels at room temperature. The finished gels were immersed in a 100%(w/v) ethanol solution for 1 hour, and distilled water was added to remove the ionic liquid by diffusion. The gels swollen in water were cut in a rectangular form with a width of 1 cm and a length of 3 cm, and then dried.

### Example 3: Tensile strength test of chitosan and chitosan composite

8 types of films (Neat, C01, C04, C07, C15, D15, C15 SP, D15 SP) prepared in Example 1 were cut in a rectangular form of 1cmX3cm, and the thickness was measured using a micrometer to nearest 0.001 mm. A tensile strength testing instrument (Instron 3340 model) was used in a young's constant extension rate mode at a strain rate of 0.5 mm/min, and a distance between the clamps of the specimen was 1 cm. Tensile strength of each sample was measured under two different environments, at a relative humidity of about 50% and in a completely wet state by immersing the sample in about 0.15 M phosphate buffered saline (pH 7.4) for a day, respectively.

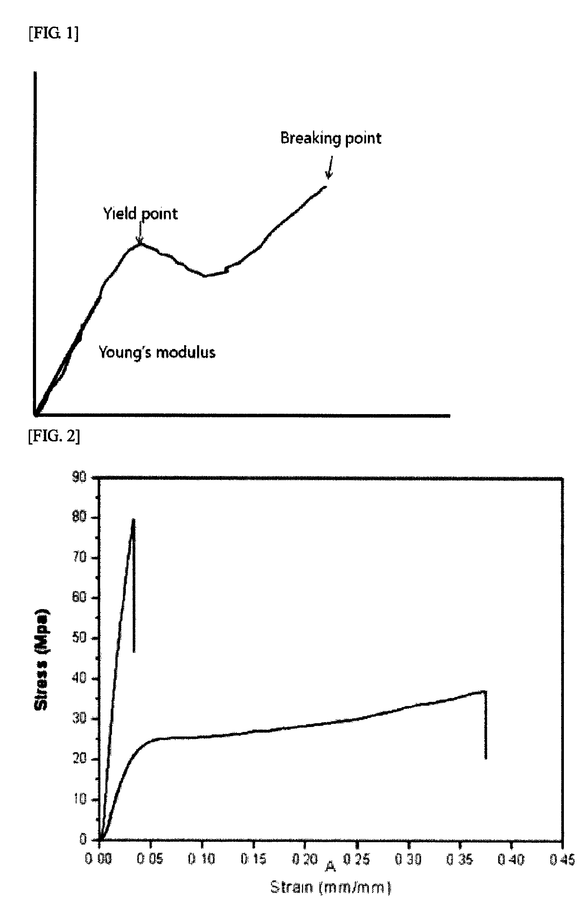

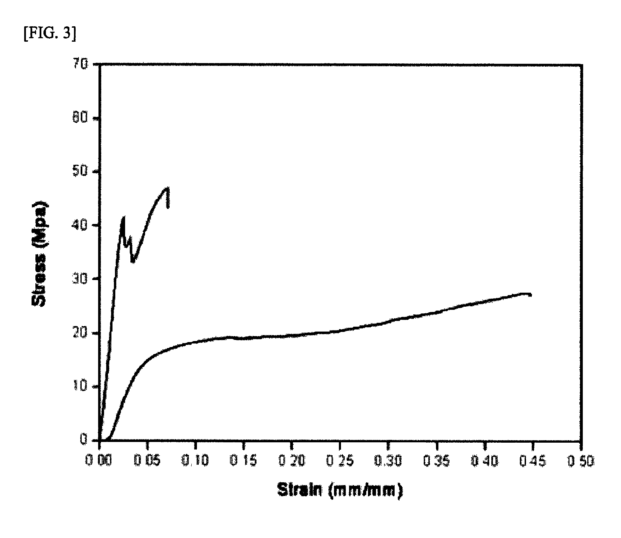

Of the samples, C15 SP and D15 SP were left in a vacuum oven at 100°C overnight (about 12 hours) so as to prepare C15 SP_annealing and D15 SP_annealing samples. Using the tensile strength testing instrument, tensile strength of each sample was measured under two different environments, at a relative humidity of about 50% and in a completely wet state by immersing the sample in about 0.15 M phosphate buffered saline (pH 7.4) for a day, respectively. The results are shown in FIGs. 2 to 3. In FIGs. 2 and 3, the black color represents physical properties of the pure chitosan film in common, the red color of FIG. 2 represents the catechol-containing chitosan composite, and the blue color of FIG. 3 represents the dopamine-containing chitosan composite. Further, average young's modulus, yield stress, yield strain stress at break (Breaking stress), and strain at break (Breaking strain) were calculated from the graph. In more detail, stress is, F(force)/A(area), a value obtained by dividing the applied force by the cross-sectional area, and the unit is N/m. In addition, strain means the ratio of extension, and is defined as the change in length/the original length. As such, the above values are obtained by applying the original length and area to the tensile strength testing instrument and operating it. First, a graph having strain on X axis and stress on y axis is obtained (see FIG. 1), in which the initial slope before the inflection point is called young's modulus, the inflection point is called a yield point. At this point, the strain is called a yield strain and the stress is called a yield stress. A point at which the sample is finally broken is called a breaking point. At this point, the strain is called a breaking strain, and the stress is called a breaking stress. The slope before fracture is called tensile strength, and is generally in proportion to the initial young's modulus. In the present invention, the young's modulus may be also used as tensile strength.

The results as obtained above are given in the following Tables 2 and 3.

**[Table 2]**

| At a relative humidity of 50% | | | | | | |
|---|---|---|---|---|---|---|
| | Ei (Mpa) | Yield stress(Mpa) | Breaking stress(Mpa) | Yield strain(%) | Breaking strain(%) | color |
| Neat chitosan | 320 | 15 | 18 | 2 | 30 | no |
| C04 | 510 | 17 | 25 | 2 | 38 | no |
| C07 | 620 | 20 | 26 | 1.0 | 41 | no |
| C15 | 800 | 15 | 36 | 1.8 | 48 | no |
| D15 | 720 | 17 | 33 | 1.9 | 45 | no |

| | | | | | | |
|---|---|---|---|---|---|---|
| (Ei: young's modulus) | | | | | | |

**[Table 3]**

| **In a completely wet state by immersing samples in 0.15 M phosphate buffered saline (pH 7.4) for a day** | | | | | | |
|---|---|---|---|---|---|---|
| | Ei (Mpa) | Yield stress(Mpa) | Breaking stress(Mpa) | Yield strain(%) | Breaking strain(%) | Color |
| Neat chitosan | 120 | 6.3 | 12.5 | 2 | 25 | no |
| C04 | 106 | 10.2 | 15.5 | 2 | 28 | no |
| C07 | 260 | 13.1 | 16.4 | 1.0 | 38 | light brown |
| C15 | 200 | 16 | 17 | 1.8 | 48 | light brown |
| D15 | 310 | 15 | 21 | 3 | 32 | no |
| C15ST | 350 | 15 | 43 | 8 | 55 | deep brom |
| D15SP | 310 | 8 | 23 | 5 | 60 | deep brown |
| C15SP_annealing | 3000 | 48 | 80 | 1 | 2.5 | deep brown |
| D15SP_annealing | 1000 | 40 | 42 | 2 | 7 | deep brown |

| | | | | | | |
|---|---|---|---|---|---|---|
| (Ei: young's modulus) | | | | | | |

As seen in Table 2, in the chitosan composite containing catechol, young's modulus (initial modulus) was increased to 800 Mpa at a relative humidity of about 50% in proportion to the catechol concentration. Sample D15 which is a chitosan composite containing dopamine also showed young's modulus of 720 Mpa. The young's modulus value of the chitosan composite sample containing catechol or dopamine was much higher than that (320 Mpa) of the neat chitosan sample containing only chitosan.

In addition, breaking strain and stress of the chitosan composite containing catechol were also higher than those of the neat chitosan sample containing only chitosan, and they were greatly increased in proportion to the catechol concentration. Breaking strain and stress of Sample D15 which is a chitosan composite containing dopamine were also increased, compared to those of the neat chitosan sample. It seems that these results are attributed to restriction of the movement of chitosan molecular chain by partial crosslinking reaction of catechol and dopa group of dopamine.

As seen in Table 3, young's modulus values of neat chitosan, C04, C07, C15, and D15 which were immersed in about 0.15 M phosphate buffered saline (pH 7.4) for a day to be completely wet were greatly reduced to about 50% or more, compared to those of the samples at a relative humidity of 50% (Table 2), which is attributed to water molecules in air that function as a plasticizer to make the material flexible. Color change of the samples C15 SP and D15 SP to brown, which did not occur before addition of sodium periodate, is attributed to oxidation of two hydroxyl groups on the catechol group to ketone groups.

Compared to C15 and D15, C15 SP and D15 SP showed no differences in young's modulus and breaking stress between those at a relative humidity of about 50% and those immersed in about 0.15 M phosphate buffered saline (pH 7.4) for a day to be completely wet, because sodium periodate promoted oxidation reaction, but did not greatly increase the next crosslinking reaction.

Compared to the neat chitosan, C15 SP_annealing and D15 SP_annealing which were obtained by heating C15 SP and D15 SP under vacuum at 100°C for 12 hours showed young's modulus values increased to about 24 times at a relative humidity of about 50% and to about 15 times in a completely wet state after being immersed in about 0.15 M phosphate buffered saline (pH 7.4) for a day. These results reflect that heat treatment accelerated the crosslinking reaction.

### Example 4: Test of tensile strength of chitosan and chitosan composite

### 4.1. Preparation of DOPA-containing chitosan composite

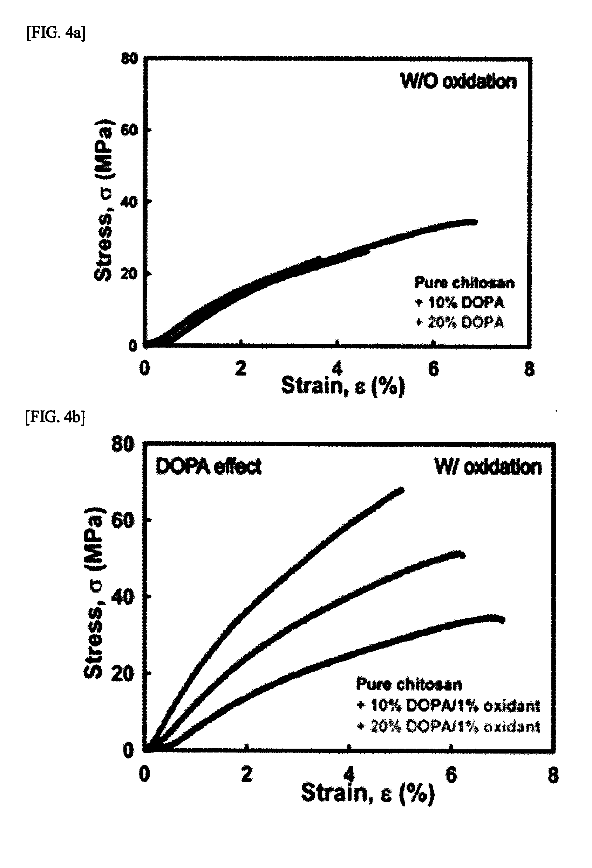

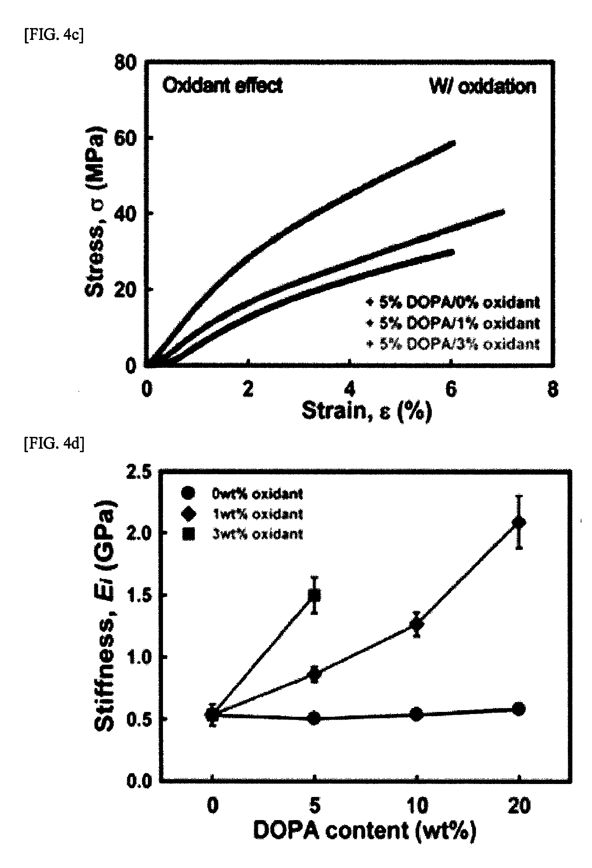

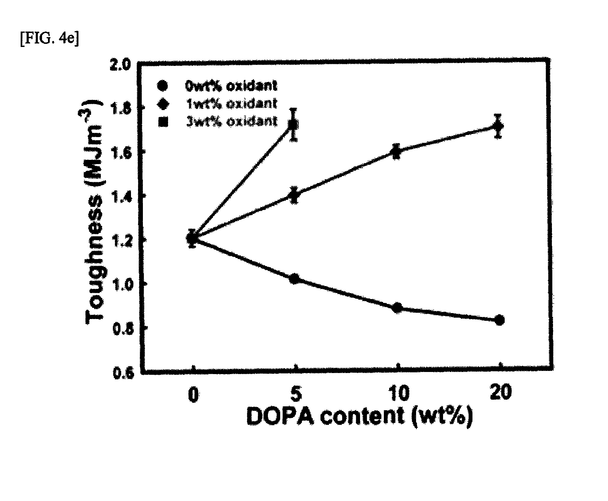

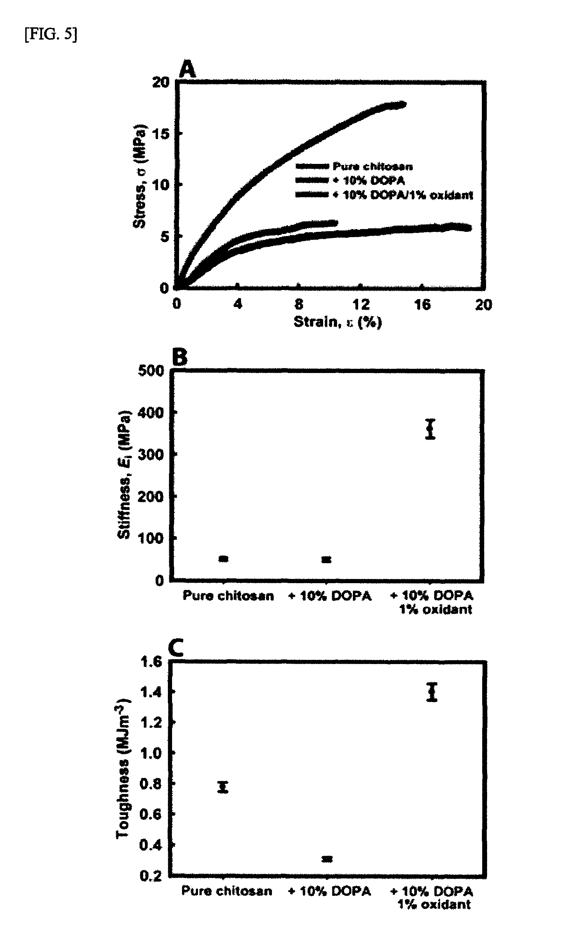

After preparation of 0.325 M acetic acid aqueous solution, 20 g of chitosan (High molecular weight, sigma-aldrich, Chitosan 419419-(Coarse ground flakes and powder) 800-2000 cP, 1 % in 1% acetic acid, Brookfield(lit.), DDA: 80% or more) was dissolved in 980 g of acetic acid aqueous solution under sonication at 40°C for 24 hours to prepare a chitosan/acetic acid aqueous solution. To 30 ml of the chitosan/acetic acid aqueous solution thus obtained, 0-20 wt% of DOPA (99%(w/w) sigma-aldrich) and 0-3wt% of sodium periodate were added to satisfy the mixing ratio conditions (see FIGs. 4 and 5), and dissolved. Each 30 ml of the prepared samples was added to a petri dish of which entire bottom was coated with Teflon tape (a separate petri dish was used for each sample). Each petri dish prepared was dried in a convection oven at 40°C for 2 days to prepare freestanding films of about 0.1 mm. To completely remove the residual acetic acid and water, the obtained films were placed in a vacuum oven at 50°C overnight.

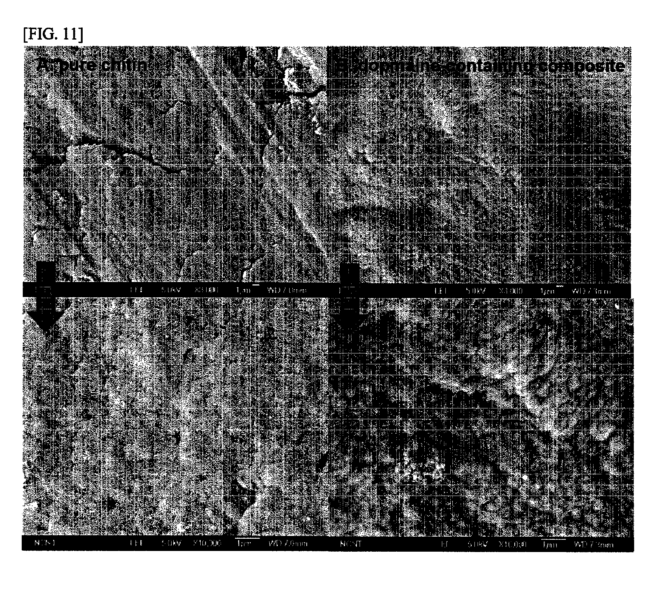

The chitosan and chitosan composite films thus prepared were cut in a rectangular form of 1cmX3cm, and the thickness was measured using a micrometer to nearest 0.001 mm. The electron microscopic images of the films produced are shown in FIG. 11.

A tensile strength testing instrument (Instron 3340 model) was used in a young's constant extension rate mode at a strain rate of 5 mm/min, and a distance between the clamps of the specimen was 1 cm. The samples to be measured in a dry state were completely dried in a vacuum oven at 120°C for 6 hours, and the samples to be measured in a wet state were immersed in about 0.15 M phosphate buffered saline (pH 7.4) for a day, and immediately taken out, followed by measurement of tensile strength.

Tensile strengths of chitosan and chitosan composite in a dry state (moisture content of about 1% or less) and in a swollen state in 0.15 M phosphate buffered saline (pH 7.4) are shown in FIGs. 4a to 4e and FIG. 5, respectively. Young's modulus of each sample was calculated by the method described in Example 3.

FIG. 4a shows tensile strength according to the DOPA content when no oxidant (sodium periodate; hereinafter, the same as above) was contained, FIG. 4b shows tensile strength according to the DOPA content when 1% by weight of the oxidant was contained, FIG. 4c shows tensile strength according to the oxidant content when 5% by weight of DOPA was contained, FIG. 4d shows stiffness according to the DOPA and oxidant contents, and FIG. 4e shows toughness according to the DOPA and oxidant contents (each value and error bar represent a mean value from 5 experiments and standard deviation, respectively). As shown in FIGs. 4a to 4e, pure chitosan showed Young's modulus of about 0.5 GPa in a dry state, which was increased up to 4-fold (about 2 GPa) in proportion to the amount of DOPA added.

A, B, and C of FIG. 5 show tensile strength, stiffness, and toughness according to DOPA and oxidant contents in a wet-swollen state, respectively (each value and error bar represent a mean value from 5 experiments and standard deviation, respectively). As shown in A of FIG. 5, pure chitosan showed Young's modulus of 0.05 GPa in a wet-swollen state, which was increased up to 7.1-fold (about 0.35 GPa) in proportion to the amount of dopamine added. Considering that human tendon and ligament have Young's modulus of 0.5 GPa and 0.2 GPa, respectively, the prepared chitosan composite can be used as a material for artificial tendon and artificial ligament.

### Example 5: Test of tensile strength of chitin and chitin composite

3 types of chitin and chitin composite films (dopamine content: 0% by weight, 5% by weight, 10% by weight) prepared in Example 2 were cut in a rectangular form of 1 cmX3cm, and the thickness was measured using a micrometer to nearest 0.001 mm. A tensile strength testing instrument (Instron 3340 model) was used in a young's constant extension rate mode at a strain rate of 5 mm/min, and a distance between the clamps of the specimen was 1 cm.

The samples in a dry state were prepared by completely drying each of them in a vacuum oven at 120°C for 6 hours, and the samples in a wet state were prepared by immersing them in distilled water for about 3 hours, and they were immediately taken out, followed by measurement of tensile strength.

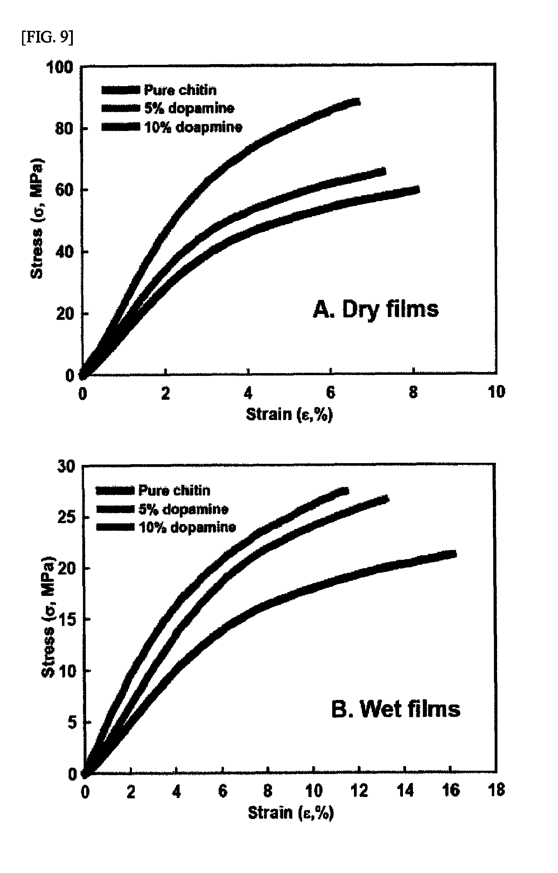

Tensile strengths of chitin and chitin composite in a dry state (top) and in a swollen state in 0.15 M phosphate buffered saline (pH 7.4) (bottom) are shown in FIG. 9. Young's modulus of each sample was calculated by the method described in Example 3. Pure chitin showed Young's modulus of about 1.5 GPa in a dry state, and Young's modulus of chitin composite was increased up to 2.1-fold in proportion to the amount of dopamine added. Pure chitin showed Young's modulus of about 0.21 GPa in a wet-swollen state, and Young's modulus of chitin composite was increased up to 2.2-fold in proportion to the amount of dopamine added. Considering that human tendon and ligament have Young's modulus of 0.5 GPa and 0.2 GPa, respectively, the chitin composite can be used as a material for artificial tendon and artificial ligament.

### Example 6: Test of water absorption and contact angle of chitin/chitosan and chitin/chitosan composite

Water absorption (EWC, equilibrium water content) of chitin/chitosan and chitin/chitosan composite prepared in Examples 1, 2, and 4.1 was measured.

Water absorption of the samples was measured as follows. The weights (Wo) of 3 types of the completely dried samples were weighed, and the samples were immersed in 0.15 M phosphate buffered saline (pH 7.4) for 3 hours, and then their weights (Wt) were weighed. The weights were measured using a scale with a minimal resolution of 0.0001. The water absorption was defined as 100X(Wₜ-W₀)/Wₜ.

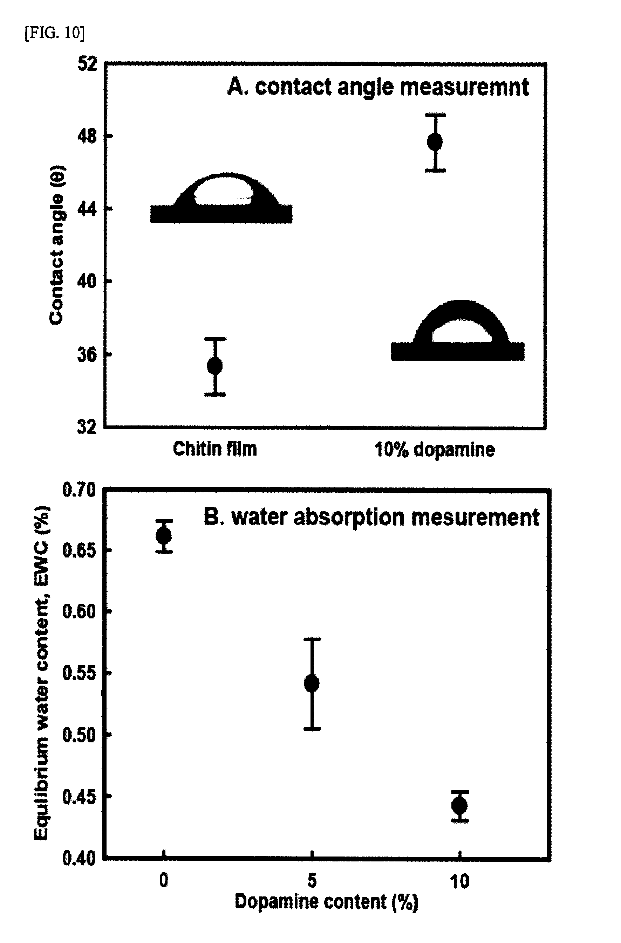

As a result, pure chitosan showed water absorption of about 66%, and chitin showed water absorption of about 65%. That of chitosan composite was reduced to about 55% in proportion to the amount of DOPA added (See Table 6), indicating that water-resistance of chitosan composite was remarkably improved, and hydrophobic melanin was produced under wet conditions. In addition, water absorption of chitin composite was reduced up to 43% within the experimental range in proportion to the amount of dopamine added (see bottom of FIG. 10)

Such reduction in water absorption is attributed to crosslinking reaction and dehydration by dopa/dopamine oxidation described above.

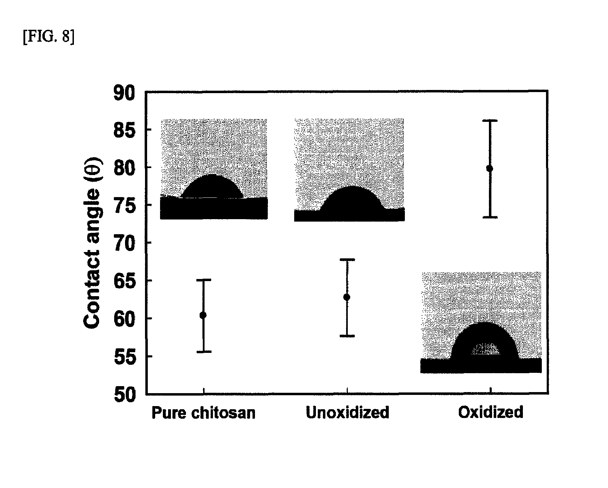

Contact angle test of chitin/chitosan and chitin/chitosan derivative films was performed. A 100 microliter drop of distilled water was dropped on the sample film prepared as flat as possible and an angle between the film and water droplet was measured to obtain a contact angle of the sample (Rutnakornpituk, et al. 2006. Carbohyd. Polym., 63(2), 229-237). The results thus obtained showed in FIGs. 8 and 10 (top). As shown in FIG. 8, the contact angle of pure chitosan was about 60 degree, and increased up to about 80 degree with addition of DOPA (unoxidized: 10wt% DOPA contained; oxidized; 10 wt% DOPA + 1 wt% sodium periodate). As shown in FIG. 10 (top), the contact angle of pure chitin was about 35 degree, and increased to about 50 degree with addition of dopamine. These results suggest that melanin layers produced by oxidation of DOPA and dopamine increased hydrophobicity of the material.

### Example 7: Quantitation of melanin present in chitin/chitosan composite

An experiment for quantifying melanin present in chitosan composite (see Example 4.1) and chitin composite (see Example 2) containing different amounts of DOPA (10 wt% and 20 wt%) was performed.

First, in order to remove materials other than melanin, 4 types of DOPA-containing composites (10wt% DOPA-containing chitin composite, 20wt% DOPA-containing composite, 10wt% DOPA-containing chitosan composite, and 20wt% DOPA-containing chitosan composite) were hydrolyzed. 70 mg of DOPA-containing chitin/chitosan composite was put in a glass ampoule, together with 3.6 ml of 6 M hydrochloric acid and 0.12 ml of phenol, and completely sealed under vacuum. The ampoules containing each sample were heated at 110°C for 48 hours. Thereafter, each sample was taken from the ampoule, and hydrochloric acid and phenol were dried using a rotary evaporator to make the sample powder. The powder samples were washed with distilled water and ethanol to remove hydrophilic hydrolysis products, thereby preparing hydrolyzed samples.

Melanin contents of the samples were quantified by hydrogen peroxide degradation (Moses, D and J. H Waite, Journal of the biological chemistry, 2006, Vol. 281, Issue 46, 34826-34832). Sepia melanin, the hydrolyzed samples prepared above, and non-hydrolyzed samples were degraded in a basic hydrogen peroxide aqueous solution, and absorbance was measured at 560 nm. Sepia melanin, isolated from squid ink, is highly pure and thus, used as a standard in various experiments. Sepia melanin was purchased from Sigma-aldrich and used.

First, absorbances of 0, 0.1, 0.2, 0.5, and 1 mgl/ml of sepia melanin degraded by hydrogen peroxide degradation were obtained. A standard curve of melanin absorbance was plotted from the results by a least square method, and compared to absorbance of 1 mg/ml of the sample solution so as to calculate the melanin content in the sample. The sample and sepia melanin were subjected to hydrogen peroxide degradation as follows. 1 volume-fold of 10 N sodium hydroxide and 2 volume-folds of 30%(w/v) hydrogen peroxide were mixed well with 37 volume-folds of water and the sample (to the sample concentration of 1 mg/ml), sealed and stored at 70°C for a day. The aqueous solution was centrifuged at 14,000 rpm to discard the residual solid impurities and absorbance of the supernatant was measured. The standard curve of absorbance at 560 nm obtained from sepia melanin was represented with a least square coefficient of R2 = 0.8767, and absorbance of the hydrolyzed sample or non-hydrolyzed sample was applied to this standard curve.

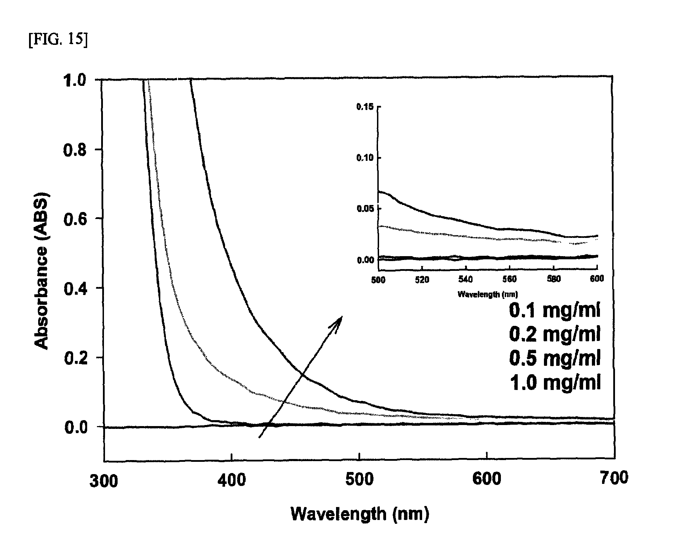

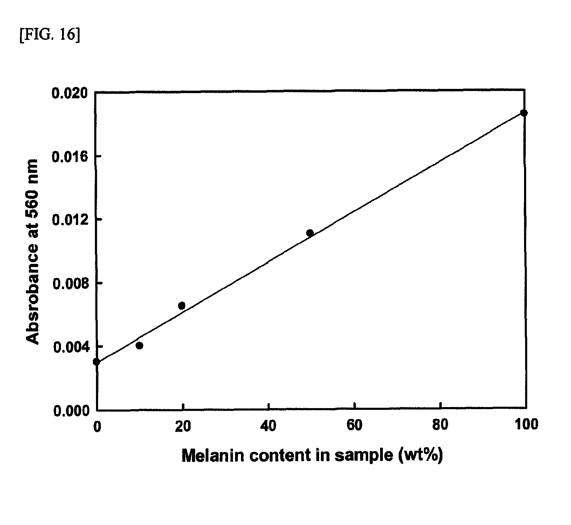

The obtained results are shown in FIGs. 15 and 16. These results showed that the non-hydrolyzed chitin/chitosan composite containing 10% or 20% DOPA contained 8.6 or 12.2 wt% of melanin, and the hydrolyzed chitin/chitosan composite containing 10% or 20% DOPA contained 94.6 wt% or 95.2 wt% of melanin. In other words, it can be seen that 90% and 84 wt% of DOPA in the non-hydrolyzed samples were oxidized to melanin. It has been known that most biomaterials including chitin and chitosan can be degraded by hydrochloric acid hydrolysis, but melanin cannot be degraded thereby, because crosslinking reaction and strong hydrophobic interaction between melanin molecules stabilize melanin. For this reason, the hydrolyzed samples showed high melanin contents.

### Example 8: Test of chitin and chitin composite in osteoblast

### Cytotoxicity of chitin and chitin composite prepared in Example 2 was examined.

In detail, mouse osteoblast (MC3T3-E1; Riken cell bank) was cultured in a 37°C incubator using 10% FBS (fetal bovine serum; Hyclone), 1% antibiotic-antimycotic (Hyclone)-containing animal cell culture medium (alpha-MEM; Hyclone). The cells were detached from a cell culture plate, and diluted at a density of 2x10⁵ cells/ml using the culture medium containing no 10% FBS. The chitin and chitin composite films were cut to the shape and size suitable for a 12-well cell culture plate (Falcon, USA), and placed therein. Then, the cells were added thereto at a density of 1x10⁵ cells/well, and incubated for 1 hour.

After incubation, the number of the living cells was counted by CCK-8 (cell counting kit-8; Dojindo, Japan) assay. First, to remove non-adherent cells after incubation, washing was performed using PBS (phosphate buffered saline; Hyclone), and 50 µl of CCK-8 solution was injected to the wells. Mitochondria in the living cells reduces 2-(2-methoxy-4-nitrophenyl)-3-(4-nitrophenyl)-5-(2,4-disulfophenyl)-2H-tetrazolium (WST-8) to water-soluble formazan. Thus, the cells were treated with CCK-8 reagent and cultured for further 3 hours, and then formazan in the medium was measured by measuring absorbance at 450 nm using a spectrophotometer.

For continuous culture, washing was performed using PBS, and 1 ml of the medium containing 10% FBS was added, followed by incubation at 37°C. Growth of the cells was measured in the same manner as adherence.

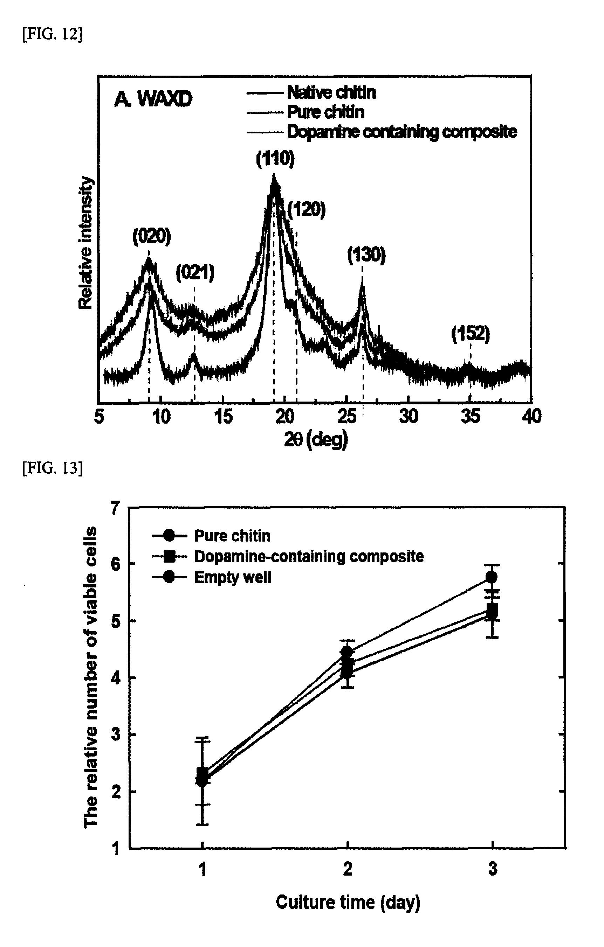

The relative numbers of living cells thus obtained are shown in FIG. 13. A relative absorbance at 450 nm of CCK medium means the relative number of living cells on a specific surface. As shown in FIG. 13, while the cells were cultured on the surface of the pure chitin film, chitin composite film, or empty well for 3 days, the relative absorbance according to culture time was compared. CCK medium on the pure chitin film, chitin composite film, or empty well showed absorbance of about 2.3, 2.2, or 2.1 which were similar within the error range on the first day, and absorbance of about 2.3, 2.2, or 2.1 on the last day. The number of living cells on chitin was lower than that of living cells on the empty well, but the difference was as small as about 10%. The difference in the number of living cells between chitin and chitin composite was as very small as less than 2%. Therefore, addition of dopamine and catechol compounds does not increase cytotoxicity.

### Example 9: TGA (thermal gravimetric analysis) of chitosan and chitosan composite

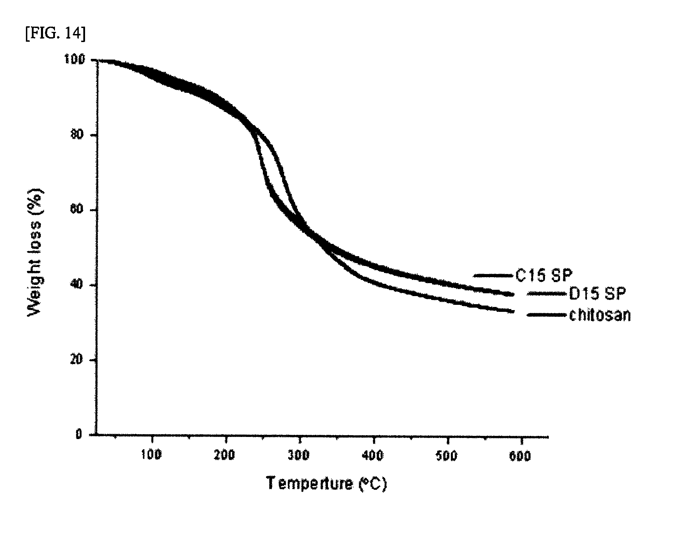

Each 5 mg of the samples prepared in Example 1, neat chitosan, C15 SP, and D15 SP was used for TGA test. TGA test was carried out using TGA (Q600, TA instrument) under nitrogen atmosphere while the temperature was raised at a rate of 10°C/min (see J. Mater. Chem., 2011, 21, 6040-6045; Facile synthesis of organo-soluble surface-grafted allsingle-layer graphene oxide as hole-injecting buffer material in organic light-emitting diodes). The results thus obtained are shown in FIG. 14.

In FIG. 14, the X-axis represents temperature, and the Y-axis represents a ratio of weight to the sample initially added. After initial addition of 5 mg, if 0.5 mg thereof is degraded at the corresponding temperature by operation, it means 90%. In the data obtained from the TGA test results of FIG. 14, the graphs of all three types of the samples showed about 7% weight loss at a temperature less than 200°C. According to the literature, the reason seems to be evaporation of water absorbed by chitosan. The 5% weight loss temperature of C15 SP or D15 SP was as high as 27 or 13 degree, which means that two composites have low water contents than pure chitosan. The relative weights of two composites at 350°C or higher were as high as about 7%, compared to pure chitosan, which means that crosslinking structures exist in the composites. Unusually, the relative weights of two composites at 200°C to 320°C were low, compared to pure chitosan, which seems to be attributed to dehydration caused by crosslinking reaction.

### Example 10: Crystal structure of chitin and chitin composite

In order to examine crystal structures of chitin and chitin composite, a wide-angle X-ray diffraction (WAXD) test was performed. This test was carried out using one of X-Ray diffractometers, D/MAX-2500/PC (Rigaku, Japan) with Ni-filtered Cu Ka radiation under experimental conditions of 40 kilovolt (40kV) and 100 milliampere (100mA). Wide-angle X-ray diffraction data were measured by increasing 4° per minute from 5° to 40°. The results are shown in FIG. 12. In FIG. 12, the black line represents native chitin, the red line represents chitin film, and the blue line represents dopamine-containing chitin composite film.

## Claims

1. A composite, comprising chitosan, chitin, or a mixture thereof; and one or more selected from the group consisting of catechol, dopamine, DOPA, and methyl catechol, wherein one or more selected from the group consisting of catechol, dopamine, DOPA, and methyl catechol are crosslinked to amine groups of chitosan, chitin, or the mixture thereof via a covalent or non-covalent bond.

2. The composite according to claim 1, wherein the composite has young's modulus of 500 to 10,000 Mpa or higher at a relative humidity of 40 to 50%, and young's modulus of 180 to 5,000 Mpa at a relative humidity of 90 to 100%.

3. The composite according to claim 1, wherein a content ratio of one or more selected from the group consisting of catechol, dopamine, DOPA, and methyl catechol is 0.1 to 30% by weight, based on the weight of chitosan, chitin, or the mixture thereof.

4. The composite according to claim 1, further comprising one or more oxidants selected from the group consisting of sodium periodate, hydrogen peroxide, sodium iodate, and sodium hydroxide.

5. The composite according to claim 4, wherein a content of the oxidant is 5 to 15% by weight, based on one or more selected from the group consisting of catechol, dopamine, DOPA, and methyl catechol.

6. The composite according to any one of claims 1 to 5, wherein the composite is treated at 80 to 120°C under vacuum for 6 to 12 hours.

7. An organic reinforcing material composition comprising the composite according to any one of claims 1 to 5.

8. The organic reinforcing material composition according to claim 7, wherein the composition is in the form of film, filament, or non-woven fabric.

9. The organic reinforcing material composition according to claim 7, wherein the composite is treated at 80 to 120°C under vacuum for 6 to 12 hours.

10. A product that is manufactured by using an organic reinforcing material composition comprising the composite of any one of claims 1 to 5.

11. The product according to claim 10, wherein the product is one or more selected from the group consisting of artificial ligament, artificial tendon, artificial dental material, artificial skin, operation suture, artificial dialysis membrane, artificial dental auxiliaries, textile for clothes, and tire cord.

12. The product according to claim 10, wherein the composite is treated at 80 to 120°C under vacuum for 6 to 12 hours.

13. A method for preparing a composite, comprising the steps of:
1) dissolving chitosan, chitin, or a mixture thereof in water, an ionic solvent or a mixture thereof; and
2) adding one or more selected from the group consisting of catechol, dopamine, DOPA, and methyl catechol to the solution thus obtained.

14. The method according to claim 13, wherein an addition amount of one or more selected from the group consisting of catechol, dopamine, DOPA, and methyl catechol is 0.1 to 30% by weight, based on the weight of chitosan, chitin, or the mixture thereof.

15. The method according to claim 13, further comprising the step of (2-1) adding one or more oxidants selected from the group consisting of sodium periodate, hydrogen peroxide, sodium iodate, and sodium hydroxide.

16. The method according to claim 15, wherein an addition amount of the oxidant is 5 to 15% by weight, based on the catechol-based compound.

17. The method according to any one of claims 13 to 16, further comprising the step of (3) treating the obtained mixture at 80 to 120°C under vacuum for 6 to 12 hours, after the step (2) or (2-1).
